# EUROPEAN PATENT SPECIFICATION

(11) **EP 2 817 099 B1**
(45) Date of publication and mention of the grant of the patent: **13.12.2017**
(21) Application number: 13739775.8
(22) Date of filing: 21.02.2013
(51) Int. Cl.: B01L 3/00, C12Q 1/56, G01N 33/86, G01N 33/49

(54) **PERFUSION DEVICE, CORRESPONDING APPARATUS USING SAID PERFUSION DEVICE AND METHOD TO ANALYZE THE THROMBOTIC-ISCHEMIC AND HEMORRHAGIC PATHOLOGY**
PERFUSIONSVORRICHTUNG, ENTSPRECHENDE VORRICHTUNG MIT DIESER PERFUSIONSVORRICHTUNG UND VERFAHREN ZUR ANALYSE DER PATHOLOGIE VON THROMBOSEN, ISCHÄMIEN UND HÄMORRHAGIEN
DISPOSITIF DE PERFUSION, APPAREIL CORRESPONDANT L'UTILISANT ET MÉTHODE PERMETTANT D'ANALYSER LA PATHOLOGIE THROMBOTIQUE-ISCHÉMIQUE ET HÉMORRAGIQUE

(30) Priority: 21.02.2012 IT UD20120028
(43) Date of publication of application: 31.12.2014
(73) Proprietor: Sedicidodici Srl, 33170 Pordenone (IT)
(72) Inventor: SELMI, Ilaria, 33077 Sacile (IT); MAZZUCCATO, Mario, 30023 Concordia Sagittaria (IT)
(74) Representative: Petraz, Gilberto Luigi
(86) International application number: PCT/IB2013/000269
(87) International publication number: WO 2013/124727

(56) References cited:
- WO-A2-2006/065739
- US-A1- 2006 003 440
- BARSTAD R M ET AL: "A PERFUSION CHAMBER DEVELOPED TO INVESTIGATE THROMBUS FORMATION AND SHEAR PROFILES IN FLOWING NATIVE HUMAN BLOOD AT THE APEX OF WELL-DEFINED STENOSES", ARTERIOSCLEROSIS AND THROMBOSIS, AMERICAN HEART ASSOCIATION, US, vol. 14, no. 12, 1 December 1994 (1994-12-01), pages 1984-1991, XP008070166, ISSN: 1049-8834

## Description

### FIELD OF THE INVENTION

The present invention concerns a perfusion device, a corresponding apparatus which uses said perfusion device and a method to analyze the thrombotic-ischemic and hemorrhagic pathology, and in particular allows the "in vitro" study of primary hemostasis and the formation of a thrombus. In particular the apparatus according to the present invention provides an analysis in video images, for example in fluorescence, of a fluid, such as a biological fluid, for example, blood, or other hematic fluids, whether they are animal or human fluids and mixtures of said fluid with additive substances, or a non-biological fluid.

### BACKGROUND OF THE INVENTION

The coagulation process is the physiological mechanism, which stops the loss of blood from damaged blood vessels and is essential to guarantee the integrity of a person in the event of hemorrhages.

Thrombosis is an unwanted formation of a hemostatic plug or a thrombus inside a blood vessel or the cardiovascular system.

As is known, the adhesive and aggregative capacity of the platelets and the formation of fibrin normally control the repair of damaged tissues. The activation of the platelets causes the formation of a mass of aggregated platelets trapped in a reticulum of fibrin, which form a plug to stop hemorrhaging.

Sometimes, the platelets can exasperate the repair process, being activated in an unsuitable manner if there is a pathological change in the hemostatic process, for example atherosclerosis which can cause a dramatic event leading to obstruction of the blood vessel: thrombosis.

A pathological hemostatic event, whether it is thrombotic-ischemic, due to a sudden lack of hematic fluid in a blood vessel, as in the case of coronaries in myocardium infarct, or hemorrhagic, is a severe problem for the cardiovascular system. These problems derive from cardiovascular alterations acquired over time, such as atherosclerosis, aneurisms, arterio venous shunts, vasculitis, anomalies of the cardiac valves, atrial fibrillation and conduction diseases, venous and arterial thromboses or other.

Moreover, these pathological events are difficult to diagnose as they are also present in subjects who in normal life have never shown any pathological occurrences and who, for example, in the event of a surgical operation, can incur serious hemorrhagic or thrombotic-ischemic problems.

To this purpose it is of fundamental importance to have, in a clinical environment, an "ex-vivo" functional test, which allows to identify potential thrombotic and hemorrhagic risks in "silent" subjects for cardiovascular pathological events.

To this end the European patent application 06819957.9 in the name of the present Applicant is known, which concerns an apparatus for the diagnosis, prognosis and pharmacological monitoring of the thrombotic-ischemic and hemorrhagic pathology in the cardio-vascular apparatus.

In particular, this known apparatus comprises a chamber, also called perfusion chamber, for the passage or flow of hematic liquids. The perfusion chamber is provided with micro-channels into which, by means of pumping means, the blood previously taken from the patient in a test tube is made to flow, suitably treated with anti-coagulants such as heparin, citrate or similar, and with fluorescent probes which act as optical markers, such as for example quinacrine, phycoerythrin, or similar.

Optical acquisition means coordinated with said markers for analyses of fluorescence acquire images relating to the development of the hemostatic processes which lead to the formation of thrombi inside the micro-channels.

A processing unit processes the images acquired and supplies indications relating to the behavior of the hemostasis of the patient.

The conformation of the perfusion chamber is of fundamental importance for the acquisition and the analysis, and to be able to carry out measurements with high degrees of reliability. The perfusion chamber is typically made of transparent material, substantially not autofluorescent, in order to allow the acquisition of images by the optical acquisition means. Its geometry must be suitable to simulate the fluid-dynamic conditions desired, such as for example the degree of sliding or shear rate, and the laminar flow.

In particular, the perfusion chamber must guarantee a high hydraulic seal, in order to preserve the fluid-dynamic conditions desired and not invalidate the reliability of the analysis.

The scientific article by Barstad et al. is known: "A perfusion chamber developed to investigate thrombus formation and shear profiles in flowing native human blood at the apex of well-defined stenoses" Arteriosclerosis and Thrombosis, American Heart Association, US, vol. 14, no. 12, 1 December 1994. The article provides a perfusion chamber formed by two parallel plates connected by screws and which has a housing to insert a support for a "cover-slip" provided with an eccentric stenosis, that is a reduction in the passage cross section of the channel of the blood flow, with reduction values up to 89%. The perfusion chamber is configured to operate in an electro-medical machine directly connected to the patient. The perfusion chamber is put in line with the blood sample taken from the patient, which is aspirated by a peristaltic roll pump located downstream. The test is carried out on the patient's native blood and the flow of blood through the stenosis causes the formation of thrombi. However, the analysis of the thrombi is not carried out in real time on the blood flow in the perfusion chamber, but is carried out after the test, by means of normal-light photo-microscopy on representative sections of the stenosis zone, in order to analyze the conformation of the thrombi that have formed. Moreover, the fluidic seal between the parallel plates of the perfusion chamber is not optimal, inasmuch as the connection by screws cannot be reliable.

The prior art document WO-A-2006/065739 describes a method and an apparatus to aggregate, display and analyze the formation of thrombi. The prior art document WO-A-2006/066008 provides the identification of the response to aspirin in a patient, using a similar apparatus to WO-A-2006/065739.

The prior art document US-A-2005/0255601 describes a cartridge and a method to determine a coagulation time based on a difference in pressure inside a capillary, which includes a transparent base plate, thick and made of rigid thermoplastic material, and a thin lid made of a sheet plastic such as Mylar®. The base plate is shaped to define a spiral capillary and is attached to the lid by means of an adhesive, which can be activated by pressure or by heat. However, the spiral configuration of the capillary might not allow a valid model of laminar flow, nor guarantee the fluidic seal in closing the base plate and the thin lid.

In the field of hybridization of nucleic acid samples, it is known from US-A-2006/003440, the use of cartridges having a chamber defined by the body of the cartridge and a plate cover, and including a pumping system for mixing a sample and lines for supplying or removing media (sample and/or reagents) from the chamber.

One purpose of the present invention is to obtain a perfusion device, an apparatus and a method which allow to carry out the complete screening of the coagulation process in a reliable way and to dynamically monitor the process of formation and stabilization of the thrombus, in order to predict, diagnose and prevent thrombotic-ischemic events which are at the base of cardiovascular pathologies.

Another purpose of the present invention is to obtain an economic device of the disposable type, while still provided with high analytical reliability, and in particular which, after use, does not need to have washed either its parts or parts of the apparatus it is coupled with, in order to carry out subsequent analyses.

Moreover another purpose is to obtain a perfusion device, which guarantees the hydraulic seal of its components.

Another purpose is to obtain an apparatus and perfect a corresponding method which allows the "in vitro" diagnosis of the thrombotic-ischemic and hemorrhagic pathology, optimizing the pharmacological monitoring of the anti-aggregating and anti-coagulant therapies performed.

Another purpose of the present invention is to obtain an apparatus which allows to acquire the coagulation process in its entirety and complexity, substantially in real time, and to analyze it, at the same time or subsequently, with a high degree of reliability and precision.

Another purpose is to perfect a method to analyze the thrombotic-ischemic and hemorrhagic pathology which is reliable and precise, and allows to monitor, acquire in real time and analyze in dynamic mode the formation of thrombi, where by thrombus we mean both complex thrombus, platelet thrombus alone, and also the fibrin that has formed.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a perfusion device according to the present invention comprises a perfusion chamber provided with at least one micro-channel, advantageously a plurality of micro-channels, in which there is at least one reactive substrate, intended for the analysis to be carried out, such as a cytoadhesive substrate, like collagen, which simulates a damaged vasal surface. A fluid is able to flow in one or more of the micro-channels provided, such as a biological fluid, like blood or other hematic fluids, whether they are animal or human fluids or mixtures of said fluids with additive substances, including possible optical markers for fluorescence analyses, or a non biological fluid.

The perfusion chamber is made of a material which allows the optical acquisition, in fluorescence and/or visible light, of suitable quality images or videos of the flow of fluid inside the at least one micro-channel by means of optical acquisition means of images or videos, typically coordinated with the possible optical markers present in the fluid flowing.

In some forms of embodiment, a transparent and substantially non autofluorescent material is used, such as for example glass or plastic, such as COC (Cyclic Olefin Copolymer), COP (Cyclo Olefin Polymer), conventional polystyrene, polycarbonate PC with high optical level. Such materials, even said super-optical materials, are selected to preferably satisfy the requisites of homogenous thickness, substantially no optical aberration, high transparency, suitability for analysis with fluorescence microscopy, in particular with negligible or no autofluorescence with respect to the quality of image desired and, real time acquisition.

Another fundamental property is the high capacity of said materials to bind the reactive substances to be used as substrate for platelet adhesion.

The perfusion chamber is therefore studied to reproduce hemostasis phenomena and processes, effectively simulating the fluid-dynamic conditions of the blood in blood vessels of different caliber.

According to the present invention, the perfusion chamber is defined by a cartridge coupled with a covering plate, between which there is at least one micro-groove, in the micrometrical size range, made open on at least one of either said cartridge or said covering plate in order to define said at least one micro-channel.

In accordance with the present invention, the cartridge comprises, integrated therewith in a single body:
- a plurality of containing elements connected to said at least one micro-channel and configured to contain a biological fluid to be analyzed and/or one or more auxiliary fluids, for example for priming and/or washing;
- a selection valve configured to selectively put in fluidic communication or connection one or the other of the containing elements with said at least one micro-channel;
- a suction pump device located downstream of said at least one micro-channel and configured to aspirate the flow of fluid through said at least one micro-channel in controlled and selectively variable fluid-dynamic conditions.

In accordance with some forms of embodiment, said cartridge comprises, integrated in a single body, a light wave guide configured to be positioned facing toward said at least one micro-channel.

In accordance with some forms of embodiment, the at least one micro-groove has a mainly rectilinear development.

In accordance with some forms of embodiment, the cartridge is provided with at least one surface or coupling surface on which, during use, a coordinated adhesion surface of the covering plate is suitable to be disposed. The at least one micro-groove is made on one or the other of said surfaces and is provided with two ends, respectively an entry end and an exit end, for the fluidic connection to a circuit to feed/discharge the fluid.

In a coordinated manner, one or other of the surfaces of the covering plate and/or the cartridge are configured to close the at least one micro-groove and to delimit the micro-channel through which the fluid to be analyzed is made to pass.

In particular, it is advantageous to provide that the surfaces on which the reactive substances are sprinkled are suitably treated to confer upon them hydrophile properties, and properties promoting the bond with the cytoadhesive substrate, such as collagen, and the repeatability of said bond. It is indeed preferable that the bond of the cytoadhesive substrate, such as collagen, is stable over time, reliable and quantifiable. It is possible to provide, for example, that said surfaces are treated with ethanol which has good degreasing capacities and renders the surface hydrophile.

According to one form of embodiment, the micro-channel is made on the surface of the cartridge and the coupling surface of the covering plate closes the micro-channel at the top.

In a variant embodiment, the two entry and exit ends are fluidically connected to said circuit by means of respective channels made at least transversely through the thickness of the cartridge.

In one form of embodiment, one of the channels, that is, the one which is directly connected to the entry end, is associated with an auxiliary pipe made in the cartridge for the introduction of additive substances into the fluid to be analyzed, which can be bio-markers, calcium or other.

In another form of embodiment, the channel which is directly connected to the entry end of the micro-groove is provided with at least one tank for mixing the fluid to be analyzed with the additive substances. In particular, the mixing tank determines a wider section which generates a turbulence which promotes the mixing of the fluid with the additive substances.

It is advantageous to provide, in some forms of embodiment, that the micro-groove, advantageously obtained by removing material, is defined by walls which extend substantially orthogonal and parallel with respect to the coupling surface of the cartridge. This solution allows to limit the effects of optical distortion and reflection which can occur during the image acquisition.

It is however quite clear that in other forms of embodiment, the micro-groove or micro-grooves can be defined by walls which extend transversely with respect to the coupling surface of the cartridge. It is indeed not excluded that the micro-grooves can have a trapezoid shaped section for example.

Advantageously, the coupling surface of the cartridge and the adhesion surface of the covering plate are substantially coplanar with respect to each other and geometrically mating to obtain a coplanar coupling thereof, both to increase the hydraulic seal of the perfusion chamber and also to reduce the optical disturbance effects during the acquisition of images.

In another form of embodiment, the cartridge is provided, at least partly integrated therein, with a suction pump device disposed downstream of the channel which is directly connected to the exit end of the micro-channel.

The suction pump device comprises at least one containing element of the fluid analyzed and a suction element provided to aspirate the fluid, making it pass through the micro-channel, and to keep the coupling surface of the cartridge and the adhesion surface of the covering plate adherent with respect to each other by means of a sucker effect which guarantees the hydraulic seal of the perfusion chamber.

According to some aspects of the present invention, the perfusion device described herein is of the disposable type and does not need washing after use, thus avoiding any condition in which the operator can come into contact with potentially infected or dangerous fluid.

In other forms of embodiment, the perfusion device comprises integrated pressure means suitable to maintain the surface of the cartridge in contact against the adhesion surface of the covering plate.

The advantage of the pressure means is that they promote the adherence of the coupling and adhesion surfaces of the cartridge and the covering plate, to obtain a hydraulic seal between the cartridge and the covering plate of the perfusion device.

For example it is possible to provide that said pressure means comprise mechanical clamping devices or magnetic elements which, through reciprocal attraction, maintain a coordinated contact between the covering plate and the cartridge.

The present invention also concerns an apparatus for dynamic analysis in real time of the thrombotic-ischemic and hemorrhagic pathology which comprises a perfusion device of the type described above.

The apparatus also comprises a circuit in which the fluid is able to flow, and a suction pump device, integrated in the perfusion device, in order to move the fluid through the circuit and the micro-channel in controlled and selectively variable fluid-dynamic conditions.

The circuit, as referred to here, is intended as closed with respect to the external environment, that is, such as to prevent any leakage or loss of fluids, which could cause contamination, or come into contact with the operator.

Optical acquisition means of images or videos are also provided, and electronic processing means suitable respectively to acquire, advantageously at high frequency, in the range of about 25-30 frames/second, and to process images of the flow moving from the at least one micro-channel of the perfusion chamber.

In some forms of embodiment, the optical acquisition means of images or videos advantageously comprise at least one optical module for fluorescence microscopy, connected in series to filming means, for example a video camera or photo camera. The filming means are, for example, but not only, of the CCD type, and have a recording/acquisition system integrated, connected to electronic processing means, such as a computerized digital system which has a computer program memorized inside it. The program, when executed in the memory of the computerized system, is able to analyze and acquire in real time the morphological, kinetic and biochemical variations inside and outside the platelets and the formation of the thrombus and its stabilization, in response to different sliding forces applied and different cytoadhesive surfaces.

According to another form of embodiment, other pressure means can be provided, suitable to press and keep pressed to each other the covering plate and the cartridge, to guarantee the hydraulic seal of the perfusion chamber.

According to another form of embodiment, the pressure means comprise a support element associated with an elastic element and a covering element. The perfusion chamber is disposed in use on the elastic element and the covering element is suitable to keep the covering plate pressed respectively against the support element and the support element pressed against the elastic element, thus absorbing possible non parallelisms between the contact surfaces and guaranteeing a substantially continuous adherence between the two surfaces of cartridge and plate.

In another form of embodiment in which the suction pump device may not be provided integrated in the perfusion device, it can be disposed downstream of the perfusion chamber and is configured to aspirate the fluid, making it pass through said at least one micro-channel.

In any case, one advantage of working under suction is that, when the suction pump device is working, it determines the suction of the covering plate against the cartridge, generating a sucker effect, which guarantees the hydraulic seal of the perfusion chamber.

With the present invention it is therefore possible to carry out a test on platelet functionality and the coagulation process in dynamic conditions, which cannot be assessed by devices existing in the state of the art. Unlike the apparatuses commonly used in the sector, the present invention allows to simulate the vascular ambient of the human or animal body and to acquire in real time the information relating to the formation of thrombi.

Indeed the present invention allows an optical or video acquisition in real time which, when processed, returns dynamic information on the test for the formation of the thrombus, unlike the end point analysis returned by the majority of diagnostic devices, which study the functionality of platelets and/or coagulation.

In order to emulate physiological conditions during the test with maximum accuracy, the shear rate of the hematic flow is advantageously known and controlled in all the micro-channels of the perfusion chamber, as is the working temperature which must be advantageously equal to the physiological temperature (37°C in the case of humans). This is particularly important and effective for monitoring anti-coagulant or anti-aggregating therapies.

Moreover with the present invention it is possible to use whole blood without the addition of substances which artificially induce aggregation. The presence of all the hematic components and the absence of any chemically induced aggregation renders the test more accurate (or nearer to the real value) and, at the same time, reduces the biological risk to which operators are subjected, since the blood sample undergoes less handling.

The present invention also concerns a method to analyze thrombotic-ischemic and hemorrhagic pathology which comprises a first step in which a fluid, such as blood, hematic or biological fluids, whether they are animal or human, and mixtures of said fluids with additive substances, is introduced and moved in controlled and variable fluid-dynamic conditions through a circuit by means of a suction pump device. In particular the biological fluid is made to pass through at least one micro-channel of a perfusion chamber in which a reactive substrate is present, intended for the analysis to be carried out, such as a cytoadhesive, able to simulate a damaged vasal surface and to reproduce hemostasis phenomena and processes. The method also comprises at least one second step of optical acquisition and processing of the images or videos acquired from the at least one micro-channel, by means of acquisition means of images or videos, and processed by electronic processing means. The perfusion chamber is advantageously made of a material which allows the optical acquisition of images or videos of the flow of hematic fluid inside the at least one micro-channel by said optical acquisition means of images or videos.

According to the present invention, during the first step the fluid is introduced into the perfusion chamber which is defined by at least one cartridge coupled to a covering plate between which there is at least one micro-groove to define said micro-channel. The micro-groove is made open on at least one of either said cartridge or said covering plate with a desired depth. The cartridge comprises, integrated therewith in a single body:
- a plurality of containing elements connected to said at least one micro-channel and configured to contain a biological fluid to be analyzed and/or one or more auxiliary fluids, for example for priming and/or washing;
- a selection valve configured to selectively put one or the other of the containing elements in fluidic communication with said at least one micro-channel;
- a suction pump device, located downstream of said at least one micro-channel.

In some forms of embodiment, the flow of biological liquid is introduced into the circuit through suction, so as to create a sucker effect between covering plate and cartridge, increasing both the adherence between a coupling surface of the cartridge and a coordinated adhesion surface of the covering plate, and also the hydraulic seal between them.

According to further aspects of the method, during the first step the covering plate is compressed by pressure means against the cartridge, making both the covering and adhesive surfaces of the cartridge and of the covering plate adhere so as to guarantee the desired hydraulic seal.

According to further aspects, before said first step where the biological fluid flows, a priming step is provided in which a solution of biologically inert liquid, typically physiological solution, is introduced through the circuit and made to pass through the micro-channel of the perfusion chamber to flood the micro-channel and the areas surrounding the micro-channel. The priming step determines the formation of a liquid sealing packing between covering plate and cartridge, in order to generate a hydraulic seal between them and to guarantee in this way that afterward, during the passage of the biological fluid through the micro-channels, the fluid remains confined inside the micro-groove with no danger of leaking from the perfusion chamber, contamination of the other micro-channels or influence on the reliability of the shear-rate measurement.

Some forms of embodiment can provide that the solution of biologically inert liquid is contained in at least one of the containing elements possibly provided in the cartridge.

The combination of the sucker effect described above with the making of a liquid packing, with the coplanarity between covering plate and cartridge and with the pressure exerted by the pressure means, allows to guarantee a high hydraulic seal, suitable for the analyses to be carried out, therefore allowing a high reliability and reproducibility of the same.

The present invention also concerns a method to diagnose the thrombotic-ischemic and hemorrhagic pathology which uses an apparatus and a method of analysis as described in the attached claims.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a schematic representation of an apparatus for analyzing the thrombotic-ischemic and hemorrhagic pathology which uses a perfusion device;
- fig. 2 is a plan schematic representation of a detail of fig. 1;
- fig. 3 is a front view of a part of fig. 2;
- fig. 4 is a view in section of fig. 2 from IV to IV;
- fig. 5 is a partly sectioned lateral view of fig. 1;
- fig. 6 is an enlarged view of a detail of fig. 1;
- fig. 7 is an enlarged detail of fig. 5;
- fig. 8 is an enlarged detail of fig. 6;
- fig. 9 is a plan view of fig. 5;
- fig. 10 is a graph of the development of the percentages of thrombi which cover the adhesion surface of the micro-channel as a function of time, in accordance with three different clinical behaviors;
- fig. 11 is a lateral view of a variant of fig. 4;
- fig. 12 is a plan view of a variant of fig. 2;
- fig. 13 is a view of another perfusion device
- fig. 14 is a perspective view of another perfusion device;
- fig. 15 is a schematic view of some forms of embodiment of a perfusion device according to the present invention;
- figs. 16 and 17 are schematic views of some forms of embodiment of a perfusion device according to the present invention in two operating conditions;
- figs. 18, 19 and 20 are schematic views of some forms of embodiment of a perfusion device according to the present invention in three operating conditions;
- figs. 21, 22 and 23 are schematic views of some forms of embodiment of a perfusion device according to the present invention in three operating conditions.

To facilitate comprehension, the same reference numbers have been used, where possible, to identify identical common elements in the drawings.

### DESCRIPTION OF SOME FORMS OF EMBODIMENT

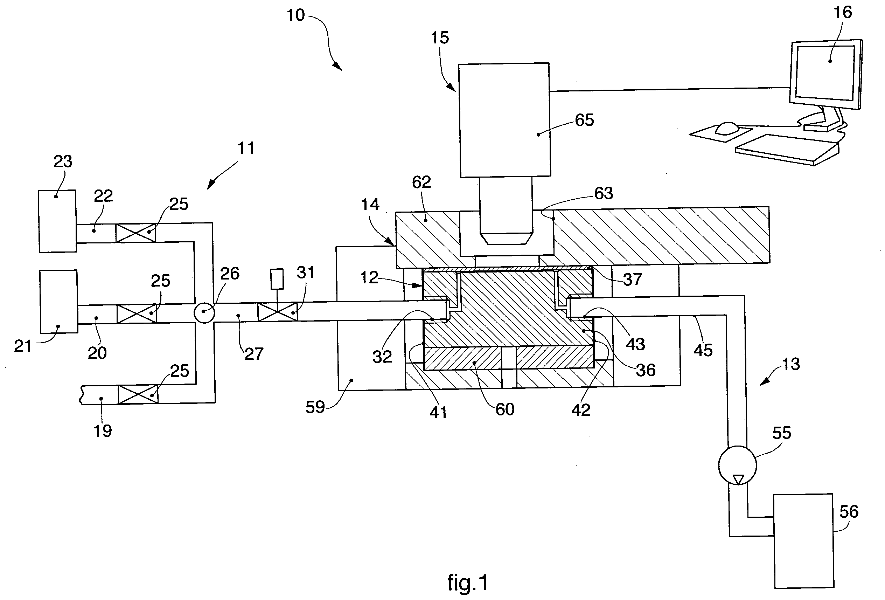

With reference to fig. 1, an apparatus for analyzing the thrombotic-ischemic and hemorrhagic pathology is indicated in its entirety by the reference number 10 and comprises a device 11 to feed the fluids to a perfusion device comprising a perfusion chamber defined by a cartridge 36 and a covering plate, or slide 37, a press 14, an optical acquisition device 15 of images or videos and a processing unit 16 to process the images acquired by the optical acquisition device 15.

The apparatus 10 may include a hydraulic circuit 13 and the fluid feed device 11 can be connected to the hydraulic circuit 13, which provides to feed the fluid into the perfusion chamber 12. The fluid feed device 11 comprises a plurality of pipes 19, 20, 21, for example a first pipe 19 to introduce the fluid to be analyzed, in the case of a test of blood coagulation, a second pipe 20 associated to a first container 21 containing a solution of biologically inert liquid, typically a physiological solution, for example, and a third pipe 22 associated to a second container 23 containing a washing solution.

The fluid to be analyzed can be biological fluid, blood, hematic fluids whether they are animal or human fluids, and mixtures of said fluid with additive substances, or a non-biological fluid.

The fluid to be analyzed, such as for example blood, can for example already be anti-coagulated and mixed with reagents useful for the analysis to be carried out, for example markers such as fluorescent probes and/or anti-fibrin antibody or possible compounds which interact with the anti-coagulation process such as calcium or similar components.

Each of the three pipes 19, 20, 22 is provided with interception valves, such as electro-valves 25, advantageously servo controlled, for the selective introduction of the fluid to be analyzed, of the physiological solution or of the washing solution to the perfusion chamber 12.

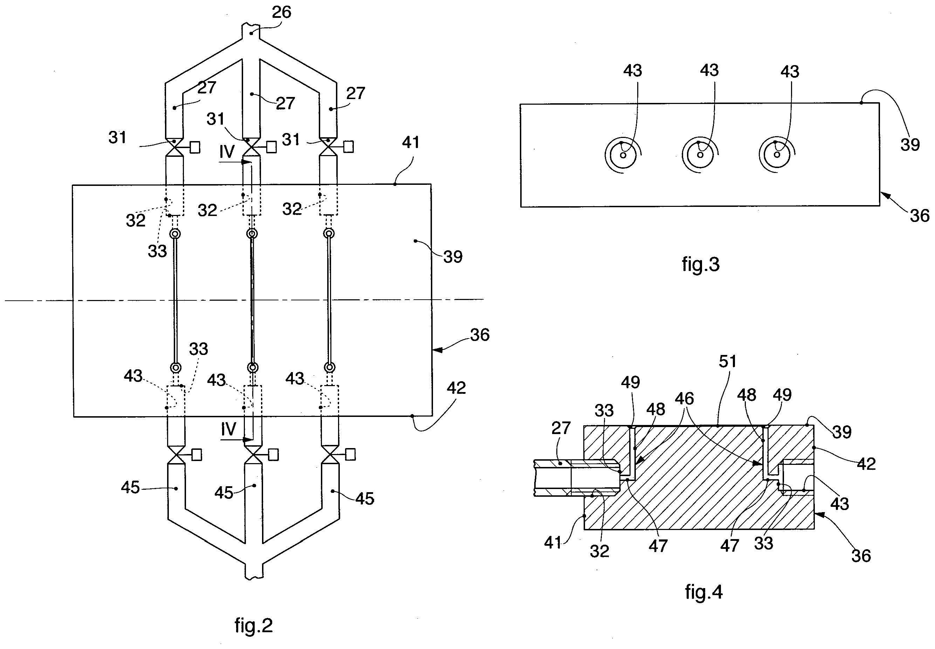

The first, second and third pipe 19, 20, 22 converge in a distribution node 26 from which three shunts 27 branch off (fig. 2).

In other forms of embodiment it is possible to provide a single pipe for the introduction both of the physiological solution and of the washing solution.

The interception valves 25 select whether the flow passes or not, and also prevent a reflux of the fluid which is being introduced from one pipe, toward the other two pipes.

Each shunt 27 is provided with an interception valve 31, like an electro-valve, controlled by the processing unit 16 to allow the selective passage through it of the blood, the physiological solution or the washing solution.

In particular each interception valve 31 is of the clamping type, made of biocompatible materials, and such as not to induce in the fluid passing through it an increase in temperature which could invalidate the analysis.

The shunts 27 connect to the perfusion chamber 12 in proximity to respective entry seatings 32, as will be described hereafter.

The perfusion chamber 12 comprises the cartridge 36, also called base element, and the slide 37, also called covering plate of the cartridge 36 and which during use is disposed in contact against a surface, hereafter called coupling surface 39 of the cartridge 36.

The cartridge 36 (figs. 2 - 4) has a substantially parallelepiped shape and is provided, in a first side 41, with said entry seatings 32 and in a second side 42, opposite the first side 41, with corresponding three exit seatings 43.

Both the entry seatings 32 and the exit seatings 43 are threaded internally: the shunts 27 and pipes 45 to discharge the fluids are connected directly and respectively to them.

The entry 32 and exit 43 seatings are made substantially blind, and have abutment surfaces 33 which end substantially orthogonal with respect to their lateral surfaces.

Moreover, the threadings inside the entry 32 and exit 43 seatings extend substantially for the entire length of the latter so that when the shunts 27 and the discharge pipes 45 are connected to the entry seatings 32 and respectively to the exit seatings 43, their ends abut against the abutment surface 33, preventing stagnation zones of the fluid to be analyzed from being generated on the bottom of the seatings, in which zones unwanted phenomena can be generated, such as the formation of clots.

This particular formation of the seatings 32 and 43 is such as to guarantee the hydraulic seal between the perfusion chamber 12 and the hydraulic circuit 13, to allow an optimal cleaning thereof after the execution of the tests, and to prevent the fluid to be analyzed from going into contact for example with metal parts or other non biocompatible materials.

In other preferential forms of embodiment, the connection between the shunts 27 and the entry 32 and exit 43 seatings are made by means of suitable connectors of the screw or snap-in type.

Channels 46 are made in proximity to the abutment surfaces 33 of the seatings 32 and 43 (fig. 4), each comprising a first segment 47 which extends substantially parallel to the direction of extension of the seatings 32 and 43, and a second segment 48 which extends substantially orthogonal to the first segment 47 and ends at the upper part toward the coupling surface 39 with a flaring 49 which opens toward the outside.

The channels 46 can have any section whatsoever and sizes of about 1 mm in diameter, even if the sizing of the channels 46 is made to prevent the formation of unwanted stagnations and accumulations of fluid, and at the same time compatible with the reductions in section of the entry channels. In particular it is necessary to prevent the flow of fluid from being obstructed because of the sudden reduction in section.

The coupling surface 39 has very restricted geometric tolerances of planarity, to cooperate effectively with the covering plate 37 so as to obtain the desired hydraulic seal.

In some forms of embodiment, one or more micro-grooves 51 are provided, with micrometric transverse sizes, for example made on the coupling surface 39, which are open at the top and are configured for the passage of the fluid.

In this case three micro-grooves 51, peripherally closed, are made inside the coupling surface 39 in correspondence to the flarings 49, and each of them connects respectively one of the entry seatings 32 with one of the corresponding exit seatings 43, in proximity to an entry end 51a and an exit end 51b. To this purpose the entry seatings 32 are disposed on the first side 41 in a position opposite the exit seatings 43 disposed on the second side 42.

The covering plate 37, disposed in contact against the coupling surface 39, closes the micro-grooves 51 defining respective closed micro-channels 54 through which during operations the fluids to be analyzed are made to pass, guaranteeing the hydraulic seal of the perfusion chamber 12.

In possible example embodiments, the micro-grooves 51 are disposed parallel with respect to each other, and can have equal lengths and widths, and possibly different depths.

In particular, the length of the micro-grooves 51 is less than that of the cartridge 36, extending for a desired partial segment of the cartridge 36 itself, so that, when the covering plate 37 is positioned, it cooperates directly and with continuity against the coupling surface 39 of the cartridge 36, obtaining the hydraulic seal on all the peripheral profile that surrounds the area where the micro-grooves 51 are made.

In some forms of embodiment, the micro-grooves 51 can have at least one main part with a rectilinear development. For example, the micro-grooves 51 can be completely rectilinear, or they can have one or more rectilinear segments and possibly curved segments. The micro-grooves 51 can be parallel with each other, or, in other example embodiments, one or more of the micro-grooves 51 can cross and intersect with respect to each other, depending on the type of phenomenon that is to be simulated.

The geometry of the individual micro-grooves 51 and the pattern which they form in the perfusion chamber 12 depends on the type of phenomenon to be simulated and on the type of test to be carried out.

The micro-grooves 51 have characteristic sizes of the micrometric type, for example depth and width different from each other and they can vary from about 100 µm to about 2000 µm.

The shape of the cross section of the three micro-grooves 51 is advantageously rectangular or square, in order to optimize the acquisition of images by the optical acquisition device 15, and together with the pressure with which the fluid to be analyzed is introduced, define the flow rate of the fluid inside the micro-grooves 51.

It is advantageous to provide that the lateral walls of the micro-grooves 51 extend substantially parallel to each other, and orthogonal to the coupling surface 39, in order to prevent reflection phenomena during the acquisition of images.

Moreover, the cross section of the micro-grooves 51 has substantially constant sizes along the whole extension, to keep the flow of fluid in a laminar flow condition and prevent phenomena of turbulence which can cause the formation of thrombi and therefore invalidate the tests. The covering plate 37 substantially consists of a thin plate with a thickness which, merely by way of example, is comprised between 0.1 mm and 4 mm, advantageously between 0.1 and 2 mm, and in any case compatible with the field depth capacity of the optical acquisition device 15. For example, the covering plate 37 can be formed by an element of the slide type, or similar or comparable.

The covering plate 37 is provided with a first face 52, or adhesion surface which, during use, is in contact against the coupling surface 39, and a second face 53 opposite the first face 52.

The first face 52, like the coupling surface 39, has a very restricted geometric tolerance of planarity, so as to render, during use, the covering plate 37 adherent to it.

Moreover, to prevent problems of reflection during the acquisition of the images, the first 52 and the second 53 face are made substantially coplanar with respect to each other.

In order to not create optical distortion effects and/or noise during the acquisition of the images, both the cartridge 36 and the covering plate 37 are made of material with a high degree of transparency and a reduced level of autofluorescence during the analytic conditions of use.

The material used to make the cartridge 36 and the covering plate or slide 37 is chosen from a group comprising glass, COC (Cyclic Olefin Copolymers), COP (Cyclo Olefin Polymers), polycarbonate with a high optical degree or similar.

In one form of embodiment, the cartridge 36 is obtained starting from a block of polycarbonate, and by means of subsequent workings by removing material, it is provided to make the entry 32 and exit 43 seatings, the channels 46 and the micro-grooves 51. The removal of material, compared to other types of working, allows to easily obtain the desired properties of planarity and coplanarity of the surfaces, of parallelism between the faces of the micro-grooves 51, as well as the desired optical properties for an accurate analysis of the tests. More specifically, the removal of material must be such as not to alter the optical properties of the material.

The hydraulic circuit 13 disposed downstream of the perfusion chamber 12 comprises at least one suction pump device 55 suitable to aspirate the fluids through the perfusion chamber 12, and a discharge tank 56 suitable to receive the fluids processed after the execution of the tests.

In particular the suction pump device 55 exerts a depression action which aspirates the fluid from the shunts 27 and makes it pass through the micro-channels 54 with a defined and controlled pressure. The suction pump device 55 not only guarantees the flow of the fluid to be analyzed but also exerts on the covering plate 37 a sucker effect which renders it adherent to the cartridge 36.

A further pressure action is exerted by the press 14 which provides to maintain the covering plate 37 under pressure against the coupling surface 39 of the cartridge 36, and therefore guarantees the hydraulic seal of the perfusion chamber 12 securely.

In some forms of embodiment, the depression action exerted by the suction pump device 55 is sufficient to keep the covering plate 37 properly adherent to the cartridge 36, and so the use of the press 14 is not necessary.

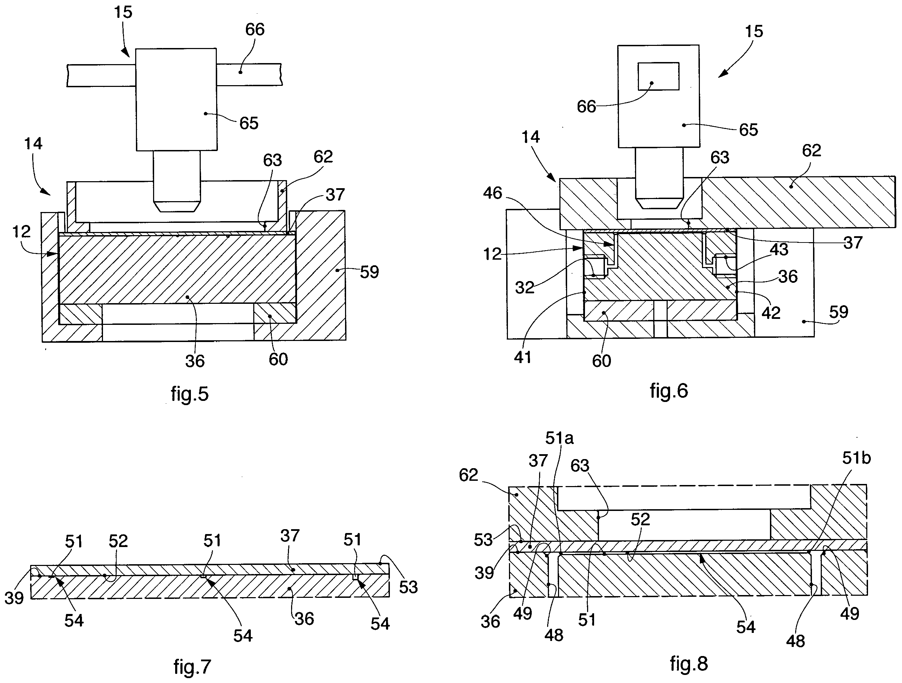

The press 14 (figs. 5 to 9) comprises a support or cartridge bearer element 59, an elastic element 60 disposed on the bottom of the cartridge bearer element 59 and on which, during use, the cartridge 36 is disposed, and a covering element 62 which, during use, is disposed in contact against the covering plate 37.

The covering element 62 comprises actuation means, not shown in the drawings, which provide to exert a pressure on the covering plate 37 and keep the latter adherent against the coupling surface 39 of the cartridge 36.

The elastic element 60 allows to compensate possible different inclinations of the cartridge 36 due to the action of the actuation means to obtain a desired parallelism between the various adhesion faces.

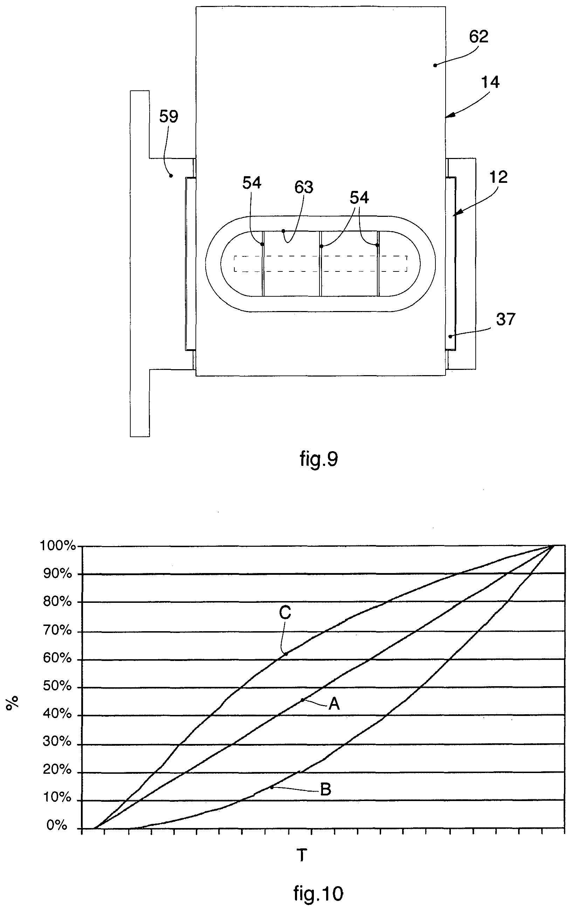

The covering element 62 (fig. 9) can be made of metal or plastic material, non-transparent to radiations, and is provided with an aperture 63 to allow the passage of luminous radiations, visible light and/or fluorescent light, to detect the images from the perfusion chamber 12.

In particular, the images are acquired substantially in the rectilinear segment, that is, in areas where the flow of fluid is not turbulent, such as for example in proximity to the flarings 49 where the fluid enters/exits from the micro-channels 54.

The optical acquisition device 15 can be a camera or a video camera associated to optical microscopy modules.

The optical acquisition device 15 can for example be an apparatus for the analysis of epifluorescence, to highlight the platelets and the fibrin marked with a suitable fluorescent probe which adheres to the cytoadhesive substrate, or an apparatus for another type of test based on a different biomarker.

The optical acquisition device 15 can comprise an optical module 65 to detect the image of the thrombi adhering on the cytoadhesive substrate, which in this case is able to slide along a guide 66 (figs. 5 and 6) to move into proximity to one of the micro-channels 54 and acquire the images.

The optical module 65 comprises sources of light radiation of the mercury, xenon or led lamps type.

In some preferential forms of embodiment, the optical module 65 comprises led lamps of the strobe type, which is selectively fed to emit light radiations only in determinate instants of time when the images are acquired. In this way the heat energy irradiated is considerably reduced and at the same time the risks of activating and coagulating the blood are reduced.

More specifically the optical module 65 is suitable to acquire a sequence of images, advantageously at high frequency in the range of about 25 - 30 frames/second, or a video film, which allow to analyze the kinetics of thrombi or hemorrhagic formation.

The processing unit 16 is suitable to process the images or the film acquired by the optical module 65 to determine the evolution of the test.

The apparatus 10 according to the present invention can advantageously comprise thermostating means to keep the perfusion chamber 12 at a desired temperature, advantageously at the physiological temperature which in the case of the human body is about 37°C.

The method for analyzing the thrombotic-ischemic and hemorrhagic pathology using an apparatus 10 as described above is described hereafter.

The method comprises at least one step in which a sample of hematic fluid is taken from a patient and suitably prepared in ways which vary depending on the tests to be obtained.

More specifically at least one sample of venous blood is required, correctly carried out, that is without hematic decantation, breakages of the veins or similar, the temporary conservation of the blood in a test tube containing anti-coagulation substances such as for example heparin, citrate or suchlike, and the addition of fluorescent substances such as quinacrine, anti-fibrin phytoerythinate antibody and, according to the type of tests, of other biomarkers or also the above biomarkers.

The apparatus 10 is in the configuration shown in fig. 1, that is, in the condition in which the perfusion chamber 12 is disposed on the cartridge bearing element 59, the press 14 keeps the covering plate 37 under pressure against the cartridge 36 and the shunts 27 and the discharge pipes 45 are connected to the cartridge 36 respectively in the entry 32 and exit 43 seatings.

The covering plate 37 and possibly also the coupling surface 39 or the micro-grooves 51 are lined with collagen substances, or cytoadhesive substances totally similar to those normally present in a damaged blood vessel. The contact between the blood and the cytoadhesive substance causes the formation and stabilization of a thrombus. The cytoadhesive substances used can be for example those cited in the European patent application 06819957.9. In this condition a preparatory step of the perfusion chamber 12 is provided, also called priming step, in which the physiological solution contained in the first container 21 is aspirated, using the suction pump device 55, through the channels 46 to fill the micro-channels 54 by capillarity and to partly flood the zones surrounding the micro-channels 54.

The physiological solution, both due to the effect of the suction action of the pumping devices 55, and also the effect of the action of the actuation devices of the press 14, both of which guarantee the adhesion of the covering plate 37 to the coupling surface 39, generates between the first face 52 of the covering plate 37 and the coupling surface 39 of the cartridge 36 a liquid packing effect which guarantees the hydraulic seal and prevents hematic contamination between one micro-channel 54 and another.

A step to introduce the fluid contained in the test tube into the perfusion chamber 12 is subsequently provided, through the first pipe 19.

The fluid is aspirated by means of the pumping devices 55 and passes through the micro-channels 54, entering into contact with the cytoadhesive substance present therein.

As soon as the platelet aggregation begins, when the blood comes into contact with the cytoadhesive substance and the platelets are activated and begin to aggregate, a step is started to acquire the images, in which the optical module 65 acquires a film and/or a sequence of images in real time, which will allow to analyze the formation kinetics of the thrombi on the evolution of the process by the processing unit 16.

More specifically the fluorescent substances introduced into the fluid are highlighted using the lamps described above, in order to supply an image which is suitably collected by the optical module 65.

The formation test of the thrombus can be carried out in visible light and in fluorescent light. However, by suitably modifying the optical module 65, the perfusion chamber 12 itself can also be used for different tests which use illumination with different wavelengths from the current ones. It is also possible to carry out a simultaneous analysis of the signal on several wavelengths.

A subsequent processing step provides that the processing unit 16, by means of dedicated software, dynamically evaluates the evolution of the platelet aggregation process and the coagulation process over time, determining two primary curves which represent the area percentage of the micro-channels 54 covered by thrombi as well as the dimension of the thrombi depending on the acquisition time.

In particular, the comparison of the curve representing the area covered by thrombi is necessary in order to discriminate, given the same area covered, the presence of many small-sized thrombi or the presence of a few large-sized thrombi, which give different clinical information.

The processing unit 16 supplies a series of data, information and/or graphs of the development of the coagulation process also as a function of the time.

Merely by way of example, fig. 10 shows a graph showing the development of the percentage of covering of platelet aggregates of the adhesion surface of the micro-channel as a function of time, in three different clinical behaviors.

In particular the curve indicated by A has a substantially rectilinear development and identifies a patient with values within the norm; curve B has a substantially exponential development and identifies a patient with a predisposition to "thrombotic" pathologies, and curve C has a substantially logarithmic development and identifies a patient with a predisposition to "hemorrhagic" pathologies.

It is clear that modifications and/or additions of parts may be made to the apparatus for the analysis of thrombotic-ischemic and hemorrhagic pathologies as described heretofore, without departing from the field and scope of the present invention.

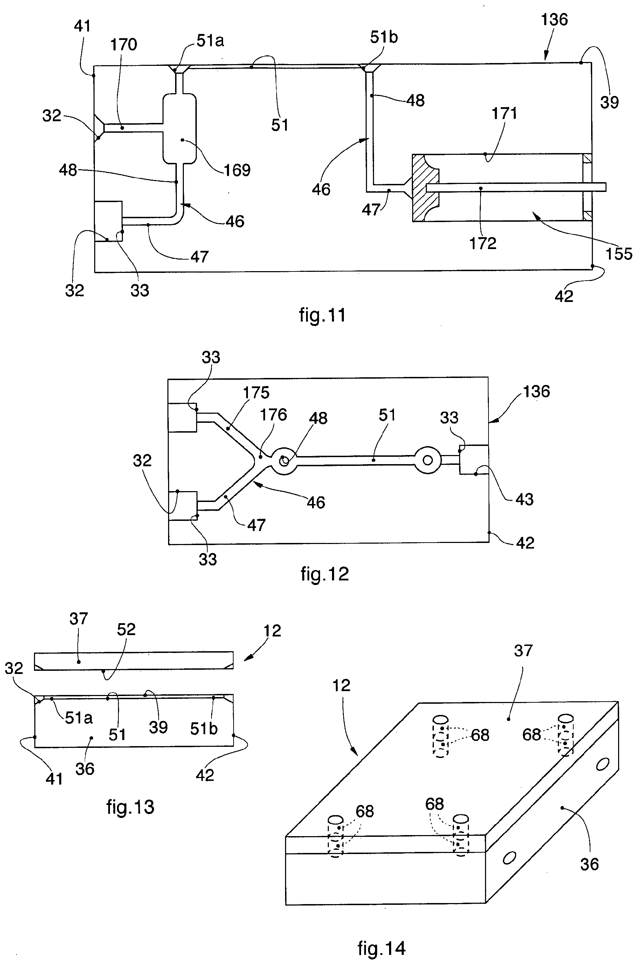

For example, fig. 11 shows another perfusion chamber 12, and in particular a cartridge 136 substantially similar to that described before with reference to cartridge 36, and also comprising a mixing tank 169 which is disposed in an intermediate position along the channel 46, directly connected to the entry end 51a of the micro-groove 51, and a suction pump device 155 integrated in the cartridge 136 and directly connected to the other channel 46.

The mixing tank 169 generates a wider section of the channel 46 and therefore a turbulence in the fluid to be analyzed, which allows to mix in additive substances which can be added through a pipe 170. The pipe 170 can be connected to the outside by means of connections similar to those provided for the entry seatings 32, or with stoppers which can be perforated by syringes with which the additive substance is introduced. The additive substances added can be biomarkers, anti-coagulant substances such as ACD or calcium solutions, which activate enzymatic reactions in the fluid to be analyzed.

On the other channel 46, instead of providing the exit seatings 43, the fluid analyzed and passed through the micro-channel or micro-channels 54, remains inside the suction pump device 155. More specifically the suction pump device 155 comprises a containing element, such as a cylindrical jacket 171, inside which a plunger 172 is made to slide, selectively sliding advantageously by means of suitable actuation means, not shown in the drawings.

By driving the plunger 172 a depression is generated inside the micro-channel or micro-channels 54, which as well as aspirating the fluid from the entry seating 32 generates a sucker effect between the coupling surface 39 of the cartridge 36, 136 and the first face 52 of the slide 37 in order to guarantee the hydraulic seal and prevent any contact between the operator and the fluid analyzed.

In other forms of embodiment (fig. 12), instead of providing the tank 169 for mixing the fluid with the additive substances, the cartridge 136 is provided with another auxiliary pipe 175 which connects with its convergence 176 in proximity to the first segment 47 of the introduction channel 46 of the fluid to be analyzed. It is therefore possible to introduce said additive substances through the auxiliary pipe 175.

In another simplified form of the perfusion chamber 12 (fig. 13), it can be provided that the micro-groove 51 is made longitudinally through its whole coupling surface 39 and that the entry 51a and exit 51b ends terminate, open toward the outside, in proximity to the sides 41, 42 of the cartridge 36. Suitable flarings or housing seatings, made in proximity to the entry 51a and exit 51b ends, allow to connect pipes to introduce/discharge the fluid.

In yet other forms instead of providing to use a press 14 to keep the slide 37 and the cartridge 36, 136 adherent to each other, it is possible to provide that pressure means are interposed between them, which exert a compression action between said two parts, at the same time guaranteeing the hydraulic seal. In another perfusion chamber (fig. 14) it can be provided that the pressure means comprise magnets 68 which are associated, advantageously in proximity to the corners of the cartridge 36, 136 and the slide 37, and disposed with respect to each other in mating positions in order to exert an attraction action between the latter two parts.

It is advantageous to combine what has been described with reference to fig. 11 with what has been described with reference to fig. 14, to make pre-packed kits, ready for use, and of the disposable type. In particular, the coupling surface 39 of the cartridge 136 and the first face 52 of the slide 37 are suitably pre-treated to make them hydrophile, on the coupling surface 39 a reactive substrate is laid, intended for the analysis to be carried out, such as cytoadhesive, in order to simulate a damaged vasal surface, and by means of the magnets 68 it is possible to couple the cartridge 136 and the slide 37 so as to constitute the perfusion chamber 12 ready for use. The perfusion chamber 12 thus obtained can be suitably packed in a controlled environment to render it available as needed, without requiring further preparation operations.

After use the perfusion chamber 12 can be suitably disposed of without requiring washing or cleaning and thus preventing the operator coming into contact with the processed fluid. Indeed the analyzed fluid remains confined inside the containing element 171 of the suction pump device 155.

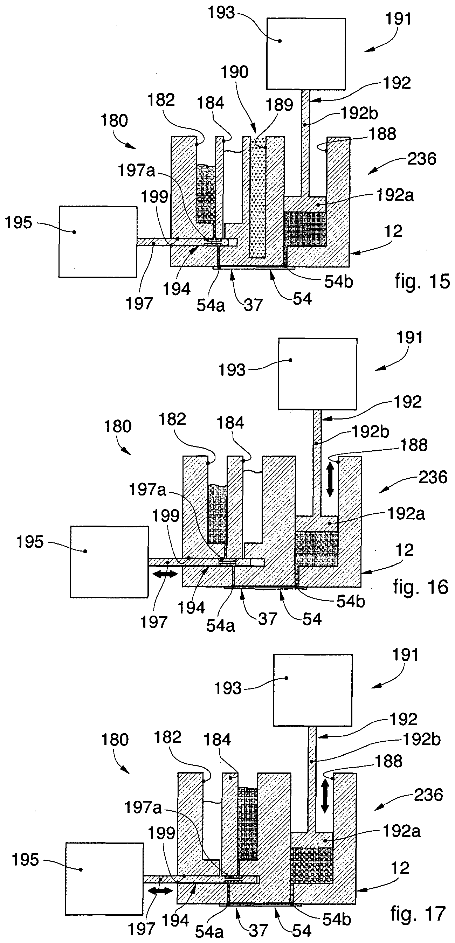

Figs. 15-23 are used to describe a plurality of forms of embodiments according to the invention of an apparatus 10 for the analysis of thrombotic-ischemic and hemorrhagic pathology which includes a perfusion chamber 12 as described here, defined by a cartridge 236 coupled to a covering plate 37. Some forms of embodiment of the apparatus 10 include a cartridge 236, similar to that described above, which comprises, in a single body, a plurality of containing elements or tanks 182, 184, 186, 188, a possible housing 189 for a possible light wave guide 190 to cooperate with the optical acquisition device 15, a suction pump device 191 comprising a plunger 192, for example a plunger of a syringe pump, insertable sliding in one of the containing elements 182, 184, 186, 188 and a selection valve 194.

For example, the containing elements 182, 184, 186, 188, like the possible housing 189 for a possible light wave guide 190, can be obtained by means of milling or similar working by removing material, or directly by molding the cartridge 236.

Advantageously, the cartridge 236 and the slide 37 coupled together constitute a perfusion device 180 which constitutes a diagnostic kit which is completely disposable. Indeed, all the elements which come into contact with the biological fluid, in particular blood, are integrated in the perfusion device 180 and, consequently, it is a finished disposable element which does not need sanitation interventions on the parts of the hydraulic-mechanical circuit of the device.

The cartridge 236 is coupled to the slide 37, as described above, in an essentially overturned configuration with respect for example to that described in figs. 1, 5-8, 13 and 14, that is, having the optical acquisition device 15 aligned with the light wave guide 190 and disposed below the perfusion device 180.

Typically, the perfusion device 180 comprises:
- a plurality of internal containing elements or tanks 182, 184, 186, 188, to store the liquids to be analyzed and the necessary auxiliary fluids, for example for priming and/or washing,
- a selection valve 194 to select the liquid to be passed through the perfusion chamber 12 defined by the cartridge 236 and by the slide 37, advantageously connected to a motor 195 which allows a linear or circular movement or a combination of the two;
- a possible light wave guide 190, to illuminate the perfusion chamber 12;
- a plunger 192, to generate a flow of liquid along the perfusion chamber 12, advantageously connected to a motor 193 which allows a linear or circular movement or a combination of the two.

Advantageously, the perfusion device 180 can be easily connected and disconnected to/from the drive shaft of each of the motors 193 and 195 which drive the selection valve 194 and the plunger 192, making the replacements rapid and safe in the light of a disposable device as above.

The perfusion device 180 formed by the cartridge 236 and slide 37 can be provided with a number of containing elements 182, 184, 186, 188, for example comprised between 1 and 5, which can be at entry to or exit from the perfusion chamber 12, according to the various functionalities required. The containing elements 182, 184, 186, 188 can contain fluids, such as blood or other biological liquids, to be analyzed, other fluids, such as blood or other biological liquids, already analyzed, reagents to be mixed inside the sample, buffer solutions needed for priming or for the test to be carried out or for washing. The perfusion device 180 provided with a plurality, for example two, three, four, five or even more than five, of containing elements 182, 184, 186, 188 allows different test configurations with a high number of reagents or auxiliary fluids, for example for priming and/or washing.

The containing elements 182, 184, 186, 188 have passageways which can be selectively connected to the one or more micro-channels 54. For example, it is possible to provide that some containing elements 182, 184, 186 are connected at exit to one or more hydraulic inlets or connections of the one or more micro-channels 54, and for example that one containing element 188 is connected at entrance to the exit of the one or more micro-channels 54.

In particular, the selection valve 194 allows to choose which of the containing elements 182, 184, 186, 188 must be fluidically connected on each occasion to the perfusion chamber 12, in particular to the one or more micro-channels 54. The selection valve 194 can be driven by means of a linear movement or a rotation.

As we said, one of the containing elements 182, 184, 186, 188 of the cartridge 236 comprises the plunger 192, sliding inside it, which allows to generate a known and constant flow of fluid, by moving the plunger 192. The containing element, for example identified by the reference number 188, which houses the plunger 192, can act at the same time as a deposit for the liquids already analyzed.

As we said, the plunger 192 can be connected to a motor 193, in particular to the corresponding drive shaft, for movement, which can be either linear or rotational. The linear movement to all purposes simulates a syringe, while a rotational movement provides that the inside of the tank, for example the tank 188, and the plunger 192, are threaded.

In some forms of embodiment, where provided, the light wave guide 190 allows the light to reach, substantially without optical aberrations, in proximity to the zone of the perfusion chamber 12, in particular of the micro-channel or micro-channels 54, where the test occurs, and allows to carry out the operations in which an illumination in the range of visible light is needed, for example a first focusing on the test micro-channel.

Fig. 15 is used to describe some forms of embodiment according to the invention, in which the perfusion device 180 includes a cartridge 236 which comprises, for example, three containing elements 182, 184, 188, of which a first containing element 182 to contain the fluid to be analyzed, a second containing element 184 for an auxiliary fluid, and a third containing element 188 to house the plunger 192, downstream of the one or more micro-channels 54, and a housing 189 is also provided for the light wave guide 190. The cartridge 236 also comprises the selection valve 194 which is formed, for example by a mobile stopper rod 197 sliding in a channel 199 made in the cartridge 236. The mobile stopper rod 197, which can be shaped with a transverse passage channel 197a, is drivable, linearly or in rotation, by a suitable motor 195, linear or rotational, and couples with the corresponding drive shaft. The channel 199 is typically positioned below the containing elements 182, 184, 186, 188, transverse to the longitudinal axis of development thereof, so as to affect the exit of each of them. For example, the channel 199 can be obtained by means of milling or similar process of removing material, or directly by molding the cartridge 236.

The plunger 192 is formed for example by a plate 192a, of a size and shape mating with those of the cross section of the third containing element 188, and by a mobile rod 192b, drivable linearly or in rotation by a suitable motor 193, linear or rotational.

Figs. 16 and 17 are used to describe forms of embodiment which can be combined with all the forms of embodiment described here, of the perfusion device 180 with three containing elements, in two operating conditions. In a first operating condition (for example see fig. 16), the selection valve 194 is driven linearly and puts the first containing element 182 in communication with the micro-channel or micro-channels 54 of the perfusion chamber 12, and in a second operating condition (for example see fig. 17), the selection valve 194 is driven linearly and puts the second containing element 184 in communication with the micro-channel or micro-channels 54 of the perfusion chamber 12, for the passage of auxiliary fluid, for example for priming and/or washing.

In some forms of embodiment, described using figs. 16 and 17, an entry 54a and an exit 54b can be provided for the micro-channel 54, or for each of the micro-channels 54. The selection valve 194 is configured to selectively put one of the exits of the containing elements 182, 184 into fluidic communication with the entry 54a of the micro-channel 54.

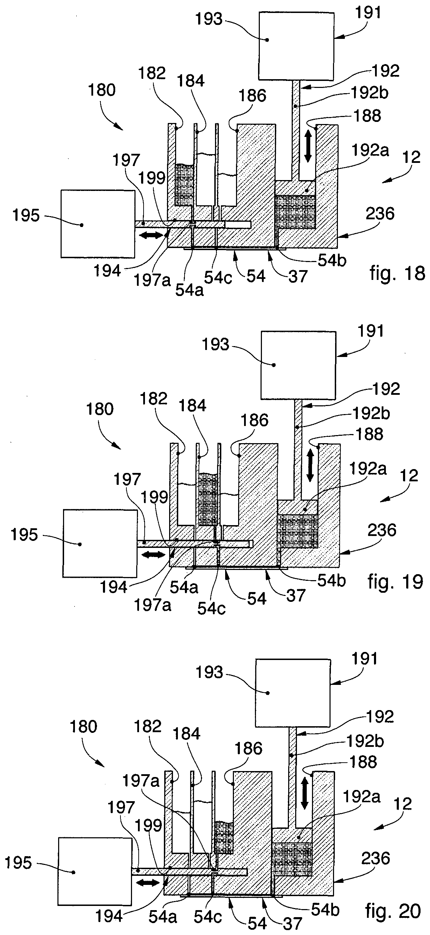

Figs. 18, 19 and 20 are used to describe forms of embodiment which can be combined with all the forms of embodiment described here, of the perfusion device 180 with four containing elements, in three operating conditions. In a first operating condition (for example see fig. 18), the selection valve 194 is driven linearly and puts the first containing element 182 in communication with the micro-channel or micro-channels 54 of the perfusion chamber 12; in a second operating condition (for example see fig. 19), the selection valve 194 is driven linearly and puts the second containing element 184 in communication with the micro-channel or micro-channels 54 of the perfusion chamber 12, for the passage of a first auxiliary fluid, reagent or additive; and in a third operating condition (for example see fig. 20), the selection valve 194 is driven linearly and puts a fourth containing element 186 in communication with the micro-channel or micro-channels 54 of the perfusion chamber 12, for the passage of a second auxiliary liquid, reagent or additive.

In forms of embodiment described using figs. 18, 19 and 20 two hydraulic entries or connections can be provided, indicated respectively by 54a and 54c, in the micro-channel 54, or for each of the micro-channels 54, including an entry for the fluid, for example blood, and an entry for one of the auxiliary fluids, for example for priming. The selection valve 194 is configured to selectively put one of the exits of the containing elements 182, 184, into fluidic communication with one or another of the entries 54a and 54c.

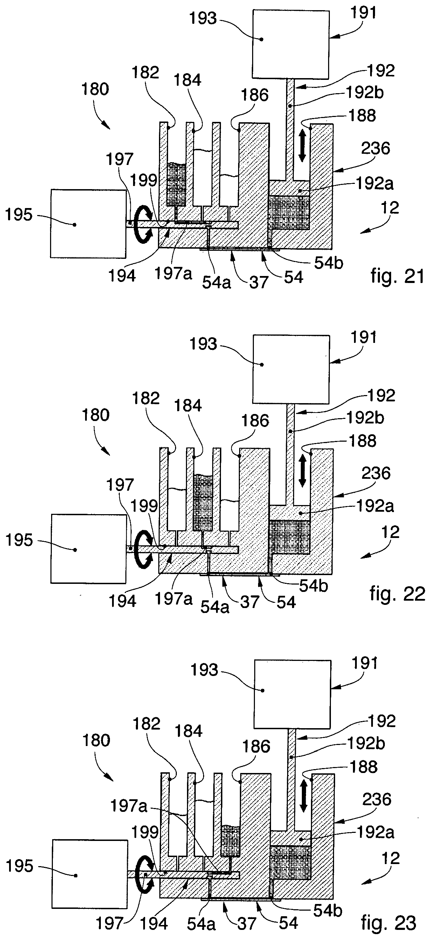

Figs. 21, 22 and 23 are used to describe forms of embodiment which can be combined with all the forms of embodiment described here, of the perfusion device 180 and similar to the forms of embodiment described using figs. 18, 19 and 20, in which, however, the selection valve 194 is driven in rotation to selectively put one of the containing elements 182, 184, 186, 188 into fluidic communication with the micro-channel or micro-channels 54 of the perfusion chamber 12. Moreover, with reference to figs. 21, 22 and 23 for example only one entry 54a and one exit 54b of the micro-channel 54 are provided, as in figs. 16 and 17.

## Claims

1. Perfusion device for the dynamic analysis of the thrombotic-ischemic and hemorrhagic pathology, comprising a perfusion chamber (12) provided with at least one micro-channel (54) configured to be connected to a feed circuit (13) of a fluid to be analyzed, in said at least one micro-channel (54) a reactive substrate being present, suitable to simulate a damaged vasal surface and to reproduce hemostasis phenomena and processes, said perfusion chamber (12) being made of a material which allows the optical acquisition, in fluorescence light and/or visible light, of images or videos of the flow of fluid inside said at least one micro-channel (54), **characterized in that** said perfusion chamber (12) is defined by a cartridge (236) coupled to a covering plate (37) between which at least one micro-groove (51), in the micrometrical size range, is provided, said at least one micro-groove (51) being made open on at least one of either said cartridge (236) or said covering plate (37) in order to define said at least one micro-channel (54); wherein said cartridge (236) comprises, integrated therewith in a single body:
- a plurality of containing elements (182, 184, 186, 188) connected to said at least one micro-channel (54) and configured to contain a biological fluid to be analyzed and/or one or more auxiliary fluids;
- a selection valve (194) configured to selectively put in fluidic communication one of the plurality of the containing elements (182, 184, 186, 188) with said at least one micro-channel (54);
- a suction pump device (191) located downstream of said at least one micro-channel (54) and configured to aspirate the flow of fluid through said at least one micro-channel (54) in controlled and selectively variable fluid-dynamic conditions.

2. Perfusion device as in claim 1, **characterized in that** said cartridge (236) comprises, integrated in a single body, a light wave guide (190) configured to be positioned facing toward said at least one micro-channel (54).

3. Perfusion device as in claim 1 or 2, **characterized in that** said at least one micro-groove (51) has a rectilinear development.

4. Perfusion device as in claim 1, 2 or 3, **characterized in that** the cartridge (236) is provided with at least one coupling surface (39) on which, during use, a coordinated adhesion surface (52) of the covering plate (37) is suitable to be disposed, **wherein** said at least one micro-groove (51) is made on said surface (39) of the cartridge (236) or on said coordinated adhesion surface (52) and is provided with two ends respectively an entry end (51a) and exit end (51b) for the fluidic connection to said feed circuit (13), wherein said adhesion surface (52) and/or said surface (39) of the cartridge (236) are configured to close said at least micro-groove (51) and to delimit said micro-channel (54).

5. Perfusion device as in claim 4, **characterized in that** said micro-groove (51) is made on said surface (39) of said cartridge (236) and said adhesion surface (52) of the covering plate (37) closes said micro-groove (51)

6. Perfusion device as in claim 5, **characterized in that** said two entry (51a) and exit (51b) ends are fluidically connected to said feed circuit (13) by respective channels (46) made at least transversely through the thickness of the cartridge (236).

7. Perfusion device as in claim 6, **characterized in that** the channel (46) which is directly connected to said entry end (51a) is associated with an auxiliary pipe (170; 175) made in said cartridge (236) for the introduction of additive substances into the fluid to be analyzed.

8. Perfusion device as in claim 7, **characterized in that** said channel (46) which is directly connected to said entry end (51a) is provided with at least one mixing tank (169) for mixing said fluid to be analyzed with said additive substances.

9. Perfusion device as in any claim from 4 to 8, **characterized in that** said micro-groove (51) is defined by walls which extend orthogonal and parallel with respect to the surface (39) of said cartridge (236).

10. Perfusion device as in any claim from 4 to 9, **characterized in that** said surface (39) of said cartridge (236) and said adhesion surface (52) of the covering plate (37) are coplanar with respect to each other.

11. Perfusion device as in any claim from 4 to 10, **characterized in that** said cartridge (236) is provided with said suction pump device (191) which suction pump device (191) is disposed downstream of the at least one micro-channel (54), **and in that** it comprises at least one containing element (188) of the fluid analyzed and a suction element (192) suitable to aspirate the fluid making it pass through said micro-channel (54), and to maintain said surface (39) of the cartridge (236) and said adhesion surface (52) of the covering plate (37) adherent with respect to each other.

12. Perfusion device as in any claim from 4 to 11, **characterized in that** it comprises integrated pressure means (68) suitable to maintain the surface (39) of the cartridge (236) in contact against the adhesion surface (52) of the covering plate (37).

13. Perfusion device as in any claim hereinbefore, **characterized in that** said suction pump device (191) comprises a suction plunger (192) positioned sliding in one containing element (188) of said containing elements (182, 184, 186, 188).

14. Apparatus for the dynamic analysis of the thrombotic-ischemic and hemorrhagic pathology comprising:
- a circuit (13) in which a fluid such as blood, hematic or biological fluids is able to flow, whether they are animal or human fluids and mixtures of said fluids with additive substances;
- a perfusion device as in any claim hereinbefore, connected to said circuit (13), wherein the suction pump device (191), of said perfusion device, is suitable to move the fluid through the circuit (13) and the perfusion device in controlled and selectively variable fluid-dynamic conditions;
- optical acquisition means (15) of images or videos and electronic processing means (16) suitable to acquire in real time and to process images of the fluid flow from said perfusion device.

15. Apparatus as in claim 14, **characterized in that** the suction pump device (191) is disposed downstream of a perfusion chamber (12) of said perfusion device and is suitable to aspirate the fluid making it pass through said micro-channel (54), and to maintain a surface (39) of the cartridge (236) and an adhesion surface (52) of the covering plate (37) adherent with respect to each other.

16. Apparatus as in claim 15, **characterized in that** it comprises pressure means (14; 68) suitable to maintain the surface (39) of the cartridge (236) in contact against the adhesion surface (52) of the covering plate (37).

17. Apparatus as in claim 16, **characterized in that** said pressure means (14) comprise a support element (59) associated with an elastic element (60) configured to accommodate the perfusion chamber (12), and a covering element (62) configured to respectively maintain said covering plate (37) pressed against said support element (59) and said support element (59) pressed against said elastic element (60).

18. Method for the dynamic analysis of the thrombotic-ischemic and hemorrhagic pathology, comprising a first step in which a fluid such as a biological fluid, such as blood or other hematic fluids, whether they are animal or human fluids and mixtures of said fluids with additive substances, or a non-biological fluid, is made to flow in controlled and variable fluid-dynamic conditions through a circuit (13) by a suction pump device (191), said fluid being made to pass through at least one micro-channel (54) of a perfusion chamber (12) in which a reactive substrate is present, intended for the analysis to be carried out, such as a cytoadhesive substrate, in order to simulate a damaged vasal surface and to reproduce hemostasis phenomena and processes, and at least one second step of optical acquisition and processing of images or videos acquired, from said at least one micro-channel (54), by optical acquisition means of images or videos (15), and processed by electronic processing means (16), and in which said perfusion chamber (12) is made of a material which allows the optical acquisition, in fluorescence light and/or visible light, of images or videos of the flow of fluid inside said at least one micro-channel (54) by means of said optical acquisition means of images or videos (15), **characterized in that** during said first step said fluid is introduced into said perfusion chamber (12) which is defined by at least one cartridge (236) coupled to a covering plate (37) between which at least one micro-groove (51) is provided to define said micro-channel (54), said micro-groove (51) being made open on at least one of either said cartridge (236) or said covering plate (37), with a desired depth, in the micro metrical size range, wherein said cartridge (236) comprises, integrated therewith in a single body:
- a plurality of containing elements (182, 184, 186, 188) connected to said at least one micro-channel (54) and configured to contain a biological fluid to be analyzed and/or one or more auxiliary fluids;
- a selection valve (194) configured to selectively put in fluidic communication one of the plurality of the containing elements (182, 184, 186, 188) with said at least one micro-channel (54);
- said suction pump device (191), located downstream of said at least one micro-channel (54).

19. Method as in claim 18, **characterized in that** the flow of fluid is introduced and moved through said at least one micro-channel (54) through suction, by means of said suction pump device (191) which also provides to maintain a surface (39) of the cartridge (236) and an adhesion surface (52) of the covering plate (37) adherent with respect to each other.

20. Method as in claim 19, **characterized in that** during said first step the covering plate (37) is compressed by pressure means (14; 68) against the cartridge (236) to make said surface (39) of the cartridge (236) and said adhesion surface (52) of the covering plate (37) adhere.

21. Method as in any claim from 18 to 20, **characterized in that** before said first step of fluid flow, a priming step is provided in which a solution of biologically inert liquid, typically physiological solution, is introduced through said circuit (13) and made to pass through said micro-channel (54) of the perfusion chamber (12) to flood said micro-channel (54) and the areas surrounding the micro-channel (54).

## Patentansprüche

1. Perfusionsvorrichtung zur dynamischen Analyse der thrombotisch-ischämischen und hämorrhagischen Pathologie, umfassend eine Perfusionskammer (12), die mit zumindest einem Mikrokanal (54) versehen ist, der dafür eingerichtet ist, um mit einem Zuführungskreislauf (13) einer zu analysierenden Flüssigkeit verbunden zu werden, wobei in dem zumindest einen Mikrokanal (54) ein reaktives Substrat vorhanden ist, das dazu geeignet ist, um eine geschädigte Gefäßoberfläche nachzuahmen und Phänomene und Vorgänge der Hämostase zu reproduzieren, wobei die Perfusionskammer (12) aus einem Material besteht, das die optische Erfassung, im Fluoreszenzlicht und/oder im sichtbaren Licht, von Bildern oder Videos des Flüssigkeitsstroms innerhalb des zumindest einen Mikrokanals (54) ermöglicht, **dadurch gekennzeichnet, dass** die Perfusionskammer (12) durch eine Kassette (236) definiert wird, die mit einer Deckplatte (37) gekoppelt ist, zwischen denen zumindest eine Mikrorille (51), im mikrometrischen Größenbereich, vorgesehen ist, wobei die zumindest eine Mikrorille (51) an zumindest einer von entweder der Kassette (236) oder der Deckplatte (37) offen ausgestaltet ist, um den zumindest einen Mikrokanal (54) zu definieren; worin die Kassette (236), in einem einzigen Körper darin integriert, umfasst:
- eine Vielzahl von Aufnahmeelementen (182, 184, 186, 188), die mit dem zumindest einen Mikrokanal (54) verbunden sind und die dafür eingerichtet sind, um eine zu analysierende biologische Flüssigkeit und/oder mehrere Hilfsflüssigkeiten aufzunehmen;
- ein Selektionsventil (194), das dafür eingerichtet ist, um eines der Vielzahl von Aufnahmeelementen (182, 184, 186, 188) mit dem zumindest einen Mikrokanal (54) selektiv in Fluidverbindung zu bringen;
- eine Saugpumpenvorrichtung (191), die stromabwärts des zumindest einen Mikrokanals (54) angeordnet ist und die dafür eingerichtet ist, den Flüssigkeitsstrom durch den zumindest einen Mikrokanal (54) unter gesteuerten und selektiv variablen fluiddynamischen Bedingungen abzusaugen.

2. Perfusionsvorrichtung nach Anspruch 1, **dadurch gekennzeichnet, dass** die Kassette (236), in einem einzigen Körper integriert, einen Lichtwellenleiter (190) umfasst, der dafür eingerichtet ist, um dem zumindest einen Mikrokanal (54) zugewandt angeordnet zu werden.

3. Perfusionsvorrichtung nach Anspruch 1 oder 2, **dadurch gekennzeichnet, dass** die zumindest eine Mikrorille (51) geradlinig verläuft.

4. Perfusionsvorrichtung nach Anspruch 1, 2 oder 3, **dadurch gekennzeichnet, dass** die Kassette (236) mit zumindest einer Kopplungsfläche (39) versehen ist, auf der, während der Benutzung, eine koordinierte Haftfläche (52) der Deckplatte (37) angeordnet werden kann, **worin** die zumindest eine Mikrorille (51) an der Fläche (39) der Kassette (236) oder an der koordinierten Haftfläche (52) ausgebildet ist und mit zwei Enden, und zwar einem Einlassende (51a) und einem Auslassende (51b), für die Fluidverbindung mit dem Zuführungskreislauf (13) versehen ist, worin die Haftfläche (52) und/oder die Fläche (39) der Kassette (236) dafür eingerichtet sind, um die zumindest eine Mikrorille (51) zu verschließen und den Mikrokanal (54) zu begrenzen.

5. Perfusionsvorrichtung nach Anspruch 4, **dadurch gekennzeichnet, dass** die Mikrorille (51) an der Fläche (39) der Kassette (236) ausgebildet ist und die Haftfläche (52) der Deckplatte (37) die Mikrorille (51) verschließt.

6. Perfusionsvorrichtung nach Anspruch 5, **dadurch gekennzeichnet, dass** die beiden Einlass- (51a) und Auslassenden (51b) mit dem Zuführungskreislauf (13) durch jeweilige Kanäle (46) in Fluidverbindung stehen, die zumindest quer durch die Dicke der Kassette (236) ausgebildet sind.

7. Perfusionsvorrichtung nach Anspruch 6, **dadurch gekennzeichnet, dass** der Kanal (46), der mit dem Einlassende (51a) direkt verbunden ist, mit einem Hilfsrohr (170; 175) angeschlossen ist, das in der Kassette (236) für die Einführung von Zusatzstoffen in die zu analysierende Flüssigkeit ausgebildet ist.

8. Perfusionsvorrichtung nach Anspruch 7, **dadurch gekennzeichnet, dass** der Kanal (46), der mit dem Einlassende (51a) direkt verbunden ist, mit zumindest einem Mischtank (169) zur Mischung der zu analysierenden Flüssigkeit mit den Zusatzstoffen versehen ist.

9. Perfusionsvorrichtung nach einem der Ansprüche 4 bis 8, **dadurch gekennzeichnet, dass** die Mikrorille (51) durch Wände definiert ist, die orthogonal und parallel zur Fläche (39) der Kassette (236) verlaufen.

10. Perfusionsvorrichtung nach einem der Ansprüche 4 bis 9, **dadurch gekennzeichnet, dass** die Fläche (39) der Kassette (236) und die Haftfläche (52) der Deckplatte (37) zueinander komplanar sind.

11. Perfusionsvorrichtung nach einem der Ansprüche 4 bis 10, **dadurch gekennzeichnet, dass** die Kassette (236) mit der Saugpumpenvorrichtung (191) versehen ist, wobei die Saugpumpenvorrichtung (191) stromabwärts des zumindest einen Mikrokanals (54) angeordnet ist, **und dass** sie zumindest ein Aufnahmeelement (188) für die analysierte Flüssigkeit und ein Saugelement (192) umfasst, das die Flüssigkeit dadurch saugen kann, indem diese den Mikrokanal (54) durchströmt, und das die Fläche (39) der Kassette (236) und die Haftfläche (52) der Deckplatte (37) aneinander haftend halten kann.

12. Perfusionsvorrichtung nach einem der Ansprüche 4 bis 11, **dadurch gekennzeichnet, dass** sie integrierte Druckmittel (68) umfasst, die dazu geeignet sind, die Fläche (39) der Kassette (236) im Kontakt gegen die Haftfläche (52) der Deckplatte (37) zu halten.

13. Perfusionsvorrichtung nach einem der vorangehenden Ansprüche, **dadurch gekennzeichnet, dass** die Saugpumpenvorrichtung (191) einen Saugkolben (192) umfasst, der in einem Aufnahmeelement (188) der Aufnahmeelemente (182, 184, 186, 188) verschiebbar angeordnet ist.

14. Apparat zur dynamischen Analyse der thrombotisch-ischämischen und hämorrhagischen Pathologie, umfassend:
- einen Kreislauf (13), in dem eine Flüssigkeit wie Blut, haematische oder biologische Flüssigkeiten fließen kann, egal ob es sich um Tier- oder Menschenflüssigkeiten oder um Mischungen der Flüssigkeiten mit Zusatzstoffen handelt;
- eine Perfusionsvorrichtung nach einem der vorangehenden Ansprüche, die mit dem Kreislauf (13) verbunden ist, worin die Saugpumpenvorrichtung (191) der Perfusionsvorrichtung dazu geeignet ist, die Flüssigkeit durch den Kreislauf (13) und die Perfusionsvorrichtung unter gesteuerten und selektiv variablen fluiddynamischen Bedingungen zu bewegen;
- optische Erfassungsmittel (15) von Bildern oder Videos und elektronische Verarbeitungsmittel (16), die dazu geeignet sind, Bilder des Flüssigkeitsstroms aus der Perfusionsvorrichtung in Echtzeit zu erfassen und zu verarbeiten.

15. Apparat nach Anspruch 14, **dadurch gekennzeichnet, dass** die Saugpumpenvorrichtung (191) stromabwärts einer Perfusionskammer (12) der Perfusionsvorrichtung angeordnet ist und dazu geeignet ist, die Flüssigkeit dadurch zu saugen, indem diese den Mikrokanal (54) durchströmt, und eine Fläche (39) der Kassette (236) und eine Haftfläche (52) der Deckplatte (37) aneinander haftend zu halten.

16. Apparat nach Anspruch 15, **dadurch gekennzeichnet, dass** er Druckmittel (14; 68) umfasst, die dazu geeignet sind, die Fläche (39) der Kassette (236) im Kontakt gegen die Haftfläche (52) der Deckplatte (37) zu halten.

17. Apparat nach Anspruch 16, **dadurch gekennzeichnet, dass** die Druckmittel (14) ein Stützelement (59), das mit einem elastischen Element (60) verbunden ist, das dafür eingerichtet ist, um die Perfusionskammer (12) unterzubringen, und ein Deckelement (62), das dafür eingerichtet ist, um die Deckplatte (37) gegen das Stützelement (59) bzw. das Stützelement (59) gegen das elastische Element (60) gedrückt zu halten, umfassen.

18. Verfahren zur dynamischen Analyse der thrombotisch-ischämischen und hämorrhagischen Pathologie, umfassend einen ersten Schritt, in dem eine Flüssigkeit wie eine biologische Flüssigkeit, wie Blut oder andere haematische Flüssigkeiten, egal ob es sich um Tier- oder Menschenflüssigkeiten oder um Mischungen der Flüssigkeiten mit Zusatzstoffen handelt, oder eine nicht-biologische Flüssigkeit dazu gebracht wird, durch einen Kreislauf (13) von einer Saugpumpenvorrichtung (191) unter gesteuerten und variablen fluiddynamischen Bedingungen durchzufließen, wobei die Flüssigkeit dazu gebracht wird, durch zumindest einen Mikrokanal (54) einer Perfusionskammer (12) durchzulaufen, in dem ein reaktives Substrat vorhanden ist, das für die durchzuführende Analyse bestimmt ist, wie ein cytoadhäsives Substrat, um eine geschädigte Gefäßoberfläche nachzuahmen und Phänomene und Vorgänge der Hämostase zu reproduzieren, und zumindest einen zweiten Schritt der optischen Erfassung und Verarbeitung von Bildern oder Videos, die, aus dem zumindest einen Mikrokanal (54), durch optische Erfassungsmittel von Bildern oder Videos erfasst und durch elektronische Verarbeitungsmittel (16) verarbeitet werden, und in dem die Perfusionskammer (12) aus einem Material besteht, das die optische Erfassung, im Fluoreszenzlicht und/oder im sichtbaren Licht, von Bildern oder Videos des Flüssigkeitsstroms innerhalb des zumindest einen Mikrokanals (54) durch die optischen Erfassungsmittel von Bildern oder Videos (15) ermöglicht, **dadurch gekennzeichnet, dass** während des ersten Schritts die Flüssigkeit in die Perfusionskammer (12) eingeführt wird, die durch zumindest eine Kassette (236) definiert wird, die mit einer Deckplatte (37) gekoppelt ist, zwischen denen zumindest eine Mikrorille (51) vorgesehen ist, um den Mikrokanal (54) zu definieren, wobei die Mikrorille (51) an zumindest einer von entweder der Kassette (236) oder der Deckplatte (37), mit einer gewünschten Tiefe, im mikrometrischen Größenbereich, offen ausgestaltet ist, worin die Kassette (236), in einem einzigen Körper darin integriert, umfasst:
- eine Vielzahl von Aufnahmeelementen (182, 184, 186, 188), die mit dem zumindest einen Mikrokanal (54) verbunden sind und die dafür eingerichtet sind, um eine zu analysierende biologische Flüssigkeit und/oder mehrere Hilfsflüssigkeiten aufzunehmen;
- ein Selektionsventil (194), das dafür eingerichtet ist, um eines der Vielzahl von Aufnahmeelementen (182, 184, 186, 188) mit dem zumindest einen Mikrokanal (54) selektiv in Fluidverbindung zu bringen;
- die Saugpumpenvorrichtung (191), die stromabwärts des zumindest einen Mikrokanals (54) angeordnet ist.

19. Verfahren nach Anspruch 18, **dadurch gekennzeichnet, dass** der Flüssigkeitsstrom durch den zumindest einen Mikrokanal (54) durch Absaugung, mittels der Saugpumpenvorrichtung (191), die auch dafür sorgt, eine Fläche (39) der Kassette (236) und eine Haftfläche (52) der Deckplatte (37) aneinander haftend zu halten, eingeführt und bewegt wird.

20. Verfahren nach Anspruch 19, **dadurch gekennzeichnet, dass** während des ersten Schritts die Deckplatte (37) durch Druckmittel (14; 68) gegen die Kassette (236) gedrückt wird, um die Fläche (39) der Kassette (236) und die Haftfläche (52) der Deckplatte (37) haften zu lassen.

21. Verfahren nach einem der Ansprüche 18 bis 20, **dadurch gekennzeichnet, dass** vor dem Schritt des Flüssigkeitsstroms ein Vorbereitungsschritt vorgesehen ist, in dem eine Lösung aus einer biologisch inerten Flüssigkeit, typischerweise einer physiologischen Kochsalzlösung, in den Kreislauf (13) eingeführt wird und dazu gebracht wird, durch den Mikrokanal (54) der Perfusionskammer (12) durchzulaufen, um den Mikrokanal (54) und die den Mikrokanal (54) umgebenden Bereiche zu überfluten.

## Revendications

1. Dispositif de perfusion pour l'analyse dynamique de la pathologie thrombotique-ischémique et hémorragique, comprenant une chambre de perfusion (12) pourvue d'au moins un microcanal (54) conçu pour être raccordé à un circuit d'alimentation (13) d'un fluide à analyser, un substrat réactif étant présent dans ledit au moins un microcanal (54), apte à simuler une surface vasculaire endommagée et à reproduire des phénomènes et des processus d'hémostase, ladite chambre de perfusion (12) étant en un matériau qui permet l'acquisition optique en lumière fluorescente et/ou en lumière visible d'images ou de vidéos de l'écoulement du fluide à l'intérieur dudit au moins un microcanal (54), **caractérisé en ce que** ladite chambre de perfusion (12) est définie par une cartouche (236) accouplée à une plaque de couverture (37), au moins une micro-rainure (51) de dimensions micrométriques étant formé entre celles-ci, ladite au moins une micro-rainure (51) étant réalisée ouverte vers au moins une de ladite cartouche (236) et de ladite plaque de couverture (37), afin de définir ledit au moins un microcanal (54) ; dans lequel ladite cartouche (236) comprend, de façon intégrée avec celle-ci d'un seul tenant :
- une pluralité d'éléments de contenant (182, 184, 186, 188) raccordés audit au moins un microcanal (54) conçu pour contenir un fluide biologique à analyser et/ou un ou plusieurs fluides auxiliaires ;
- une soupape de sélection (194) conçue pour mettre sélectivement en communication fluidique un de la pluralité des éléments de contenant (182, 184, 186, 188) avec ledit au moins un microcanal (54) ;
- un dispositif de pompe d'aspiration (191) situé en aval dudit au moins un microcanal (54) et conçu pour aspirer le fluide s'écoulant dans ledit au moins un microcanal (54) en conditions de dynamique du fluide contrôlées et sélectivement variables.

2. Dispositif de perfusion selon la revendication 1, **caractérisé en ce que** ladite cartouche (236) comprend, de façon intégrée d'un seul tenant, un guide d'ondes lumineuses (190) conçu pour être positionné faisant face audit au moins micro canal (54).

3. Dispositif de perfusion selon la revendication 1 ou 2, **caractérisé en ce que** ladite au moins une micro-rainure (51) à un développement rectiligne.

4. Dispositif de perfusion selon la revendication 1, 2 ou 3, **caractérisé en ce que** la cartouche (236) est pourvue d'au moins une surface d'accouplement (39) sur laquelle, pendant l'emploi, peut se disposer une surface d'adhésion coordonnée (52) de la plaque de couverture (37), dans lequel ladite au moins une micro-rainure (51) est réalisée sur ladite surface (39) de la cartouche (236) ou sur ladite surface d'adhésion coordonnée (52) et est pourvue de deux extrémités, respectivement une extrémité d'entrée (51a) et une extrémité de sortie (51b) pour le raccord fluidique auxdits circuits d'alimentation (13), dans lequel ladite surface adhésion (52) et/ou ladite surface (39) de la cartouche (236) sont conçues pour fermer ladite au moins micro-rainure (51) et pour délimiter ledit microcanal (54).

5. Dispositif de perfusion selon la revendication 4, **caractérisé en ce que** la dite micro-rainure (51) est réalisée sur ladite surface (39) de ladite cartouche (236) et ladite surface d'adhésion (52) de la plaque de couverture (37) ferme la dite micro-rainure (51).

6. Dispositif de perfusion selon la revendication 5, **caractérisé en ce que** lesdites deux extrémités d'entrée (51a) et de sortie (51b) sont en raccord fluidique avec ledit circuit d'alimentation (13) par des canaux (46) respectifs formés en direction au moins transversale sur l'épaisseur de la cartouche (236).

7. Dispositif de perfusion selon la revendication 6, **caractérisé en ce que** le canal (46) qui est directement raccordé à ladite extrémité d'entrée (51a) est associé à un tuyau auxiliaire (170 ; 175) réalisé dans ladite cartouche (236) pour l'introduction de substances additives dans le fluide à analyser.

8. Dispositif de perfusion selon la revendication 7, **caractérisé en ce que** ledit canal (46) qui est directement raccordé à ladite extrémité d'entrée (51a) est pourvu d'au moins un réservoir de mélange (169) apte à mélanger ledit fluide à analyser avec lesdites substances additives.

9. Dispositif de perfusion selon n'importe laquelle des revendications 4 à 8, **caractérisé en ce que** ladite micro-rainure (51) est définie par des parois qui s'étendent dans une direction orthogonale et parallèle par rapport à la surface (39) de ladite cartouche (236).

10. Dispositif de perfusion selon n'importe laquelle des revendications 4 a 9, **caractérisé en ce que** ladite surface (39) de ladite cartouche (236) et ladite surface d'adhésion (52) de la plaque de couverture (37) sont coplanaires.

11. Dispositif de perfusion selon n'importe laquelle des revendications 4 à 10, **caractérisé en ce que** ladite cartouche (236) est pourvue dudit dispositif de pompe d'aspiration (191), ledit dispositif de pompe d'aspiration (191) étant situé en aval dudit au moins un microcanal (54), et **en ce qu'**il comprend au moins un élément de contenant (188) pour le fluide analysé et un élément d'aspiration (192) apte à aspirer le fluide pour lui faire traverser ledit microcanal (54), et à maintenir ladite surface (39) de la cartouche (236) et ladite surface d'adhésion (52) de la plaque de couverture (37) adhérentes l'une à l'autre.

12. Dispositif de perfusion selon n'importe laquelle des revendications 4 à 11, **caractérisé en ce qu'**il comprend des moyens de pression intégrés (68) aptes à maintenir la surface (39) de la cartouche (236) en contact avec la surface d'adhésion (52) de la plaque de couverture (37).

13. Dispositif de perfusion selon n'importe laquelle des revendications précédentes, **caractérisé en ce que** ledit dispositif de pompe d'aspiration (191) comprend un piston d'aspiration (192) positionné coulissant dans un élément de contenant (188) desdits éléments de contenant (182, 184, 186, 188).

14. Appareil pour l'analyse dynamique de la pathologie thrombotique-ischémique et hémorragique, comprenant :
- un circuit (13) dans lequel s'écoulent un fluide comme le sang, des fluides hématiques ou biologiques, de nature animale ou humaine et des mélanges desdits fluides avec des substances additives ;
- un dispositif de perfusion selon n'importe laquelle des revendications précédentes, raccordé audit circuit (13), dans lequel le dispositif de pompe d'aspiration (191) dudit dispositif de perfusion est apte à déplacer le fluide dans le circuit (13) et dans le dispositif de perfusion dans des conditions de dynamique du fluide contrôlées et sélectivement variables ;
- des moyens d'acquisition optique (15) d'images ou de vidéos et des moyens de traitement électronique (16) aptes à acquérir en temps réel et à traiter des images de l'écoulement du fluide dudit dispositif de perfusion.

15. Appareil selon la revendication 14, **caractérisé en ce que** le dispositif de pompe d'aspiration (191) est situé en aval d'une chambre de perfusion (12) dudit dispositif de perfusion et est apte à aspirer le fluide, pour lui faire traverser ledit microcanal (54), et à maintenir une surface (39) de la cartouche (236) et une surface d'adhésion (52) de la plaque de couverture (37) adhérentes l'une à l'autre.

16. Appareil selon la revendication 15, **caractérisé en ce qu'**il comprend des moyens de pression (14 ; 68) aptes à maintenir la surface (39) de la cartouche (236) en contact avec la surface d'adhésion (52) de la plaque de couverture (37).

17. Appareil selon la revendication 16, **caractérisé en ce que** lesdits moyens de pression (14) comprennent un élément support (59) associé à un élément élastique (60) conçu pour accueillir la chambre de perfusion (12), et un élément de couverture (62) conçu pour maintenir respectivement ladite plaque de couverture (37) pressée contre ledit élément support (59) et ledit élément support (59) pressé contre ledit élément élastique (60).

18. Procédé pour l'analyse dynamique de la pathologie thrombotique-ischémique et hémorragique, comprenant une première étape dans laquelle un fluide, tel un fluide biologique, comme le sang ou d'autres fluides hématiques, d'origine animale ou humaine, et des mélanges desdits fluides avec des substances additives, ou un fluide non-biologique, s'écoulent dans des conditions de dynamique du fluide contrôlées et variables dans un circuit (13) grâce à un dispositif de pompe d'aspiration (191), ledit fluide étant mis en condition de passer par au moins un microcanal (54) d'une chambre de perfusion (12) dans laquelle se trouve un substrat réactif, destiné à permettre l'analyse, tel un substrat cytoadhésif, afin de simuler une surface vasculaire endommagée et à reproduire des phénomènes et des processus d'hémostase, et au moins une deuxième étape d'acquisition optique et de traitement d'images ou de vidéos acquis dudit au moins un microcanal (54), par le biais de moyens d'acquisition optique d'images ou de vidéos (15), et traités par des moyens de traitement électronique (16), et dans lequel ladite chambre de perfusion (12) est en un matériau qui permet l'acquisition optique en lumière fluorescente et/ou en lumière visible d'images ou de vidéos de l'écoulement du fluide à l'intérieur dudit au moins un microcanal (54) par lesdits moyens d'acquisition optique d'images ou de vidéos (15), **caractérisé en ce que** ladite chambre de perfusion (12), qui est définie par une cartouche (236) accouplée à une plaque de couverture (37), au moins une micro-rainure (51) étant formée entre celles-ci pour définir ledit microcanal (54), ladite micro-rainure (51) étant réalisée ouverte vers au moins une de ladite cartouche (236) et de ladite plaque de couverture (37), avec une profondeur souhaitée de dimension micrométrique, dans lequel ladite cartouche (236) comprend, de façon intégrée avec celle-ci d'un seul tenant :
- une pluralité d'éléments de contenant (182, 184, 186, 188) raccordés audit au moins un microcanal (54) conçu pour contenir un fluide biologique à analyser et/ou un ou plusieurs fluides auxiliaires ;
- une soupape de sélection (194) conçue pour mettre sélectivement en communication fluidique un de la pluralité des éléments de contenant (182, 184, 186, 188) avec ledit au moins un microcanal (54) ;
- ledit dispositif de pompe d'aspiration (191), situé en aval de ladite au moins une microcanal (54).

19. Procédé selon la revendication 18, **caractérisé en ce que** l'écoulement de fluide est introduit et déplacé dans ledit au moins un microcanal (54) par aspiration, au moyen dudit dispositif de pompe d'aspiration (191), qui assure également le maintien d'une surface (39) de la cartouche (236) et d'une surface d'adhésion (52) de la plaque de couverture (37) adhérentes l'une à l'autre.

20. Procédé selon la revendication 19, **caractérisé en ce que,** pendant ladite première étape, la plaque de couverture (37) est comprimée par des moyens de pression (14 ; 68) contre la cartouche (236) pour faire adhérer ladite surface (39) de la cartouche (236) et ladite surface d'adhésion (52) de la plaque de couverture (37) l'une contre l'autre.

21. Procédé selon n'importe laquelle des revendications 18 à 20, **caractérisé en ce qu'**une étape d'amorçage est mise en oeuvre, avant ladite première étape d'écoulement de fluide, dans laquelle une solution de liquide biologiquement inerte, typiquement une solution physiologique, est introduite dans ledit circuit (13) et passe par ledit microcanal (54) de la chambre de perfusion (12) pour inonder ledit microcanal (54) et les zones autour du microcanal (54).
